# EUROPÄISCHE PATENTSCHRIFT

(11) **EP 1 749 096 B1**
(45) Veröffentlichungstag und Bekanntmachung des Hinweises auf die Patenterteilung: **17.07.2013**
(21) Anmeldenummer: 04816287.9
(22) Anmeldetag: 28.12.2004
(51) Int. Cl.: C12N 15/85

(54) **HERSTELLUNGSVERFAHREN GEEIGNETER DNA-KONSTRUKTE ZUR SPEZIFISCHEN HEMMUNG DER GENEXPRESSION DURCH RNA-INTERFERENZ**
METHOD FOR THE PRODUCTION OF SUITABLE DNA CONSTRUCTS FOR SPECIFIC INHIBITION OF GENE EXPRESSION BY RNA INTERFERENCE
PROCEDE DE PRODUCTION D'ADN DE SYNTHESE APPROPRIES POUR L'INHIBITION SPECIFIQUE DE L'EXPRESSION GENIQUE PAR INTERFERENCE ARN

(30) Priorität: 28.05.2004 DE 102004026734
(43) Veröffentlichungstag der Anmeldung: 07.02.2007
(73) Patentinhaber: Mologen AG, 14195 Berlin (DE)
(72) Erfinder: SCHROFF, Matthias, 14195 Berlin (DE)
(74) Vertreter: Tegethoff, Sebastian
(86) Internationale Anmeldenummer: PCT/DE2004/002838
(87) Internationale Veröffentlichungsnummer: WO 2005/116223

(56) Entgegenhaltungen:
- WO-A-98/21322
- WO-A-2004/027063
- BRUMMELKAMP T R ET AL: "A system for stable expression of short interfering RNAs in mammalian cells" SCIENCE, AMERICAN ASSOCIATION FOR THE ADVANCEMENT OF SCIENCE,, US, Bd. 296, Nr. 5567, 2002, Seiten 550-553, XP002225638 ISSN: 0036-8075
- SCHAKOWSKI F ET AL: "A NOVEL MINIMAL-SIZE VECTOR (MIDGE) IMPROVES TRANSGENE EXPRESSION IN COLON CARCINOMA CELLS AND AVOIDS TRANSFECTION OF UNDESIRED DNA" MOLECULAR THERAPY, ACADEMIC PRESS, SAN DIEGO, CA,, US, Bd. 3, Nr. 5, PART 1, Mai 2001 (2001-05), Seiten 793-800, XP001100620 ISSN: 1525-0016
- WALL NATHAN R ET AL: "Small RNA: can RNA interference be exploited for therapy?" LANCET, XX, XX, Bd. 362, Nr. 9393, 25. Oktober 2003 (2003-10-25), Seiten 1401-1403, XP002330367 ISSN: 0140-6736

### SEQUENCE LISTING

<110> Mologen AG
<120> Herstellungsverfahren geeigneter DNA-Konstrukte zur spezifischen Hemmung der Genexpression durch RNA-Interferenz
<130> XI 774-04
<150> DE 10 2004 026 734.0
   <151> 2004-05-28
<160> 5
<170> PatentIn version 3.3
<210> 1
   <211> 47
   <212> DNA
   <213> Artificial
<220>
   <223> synthetisches Oligodesoxynukleotid
<220>
   <221> misc_feature
   <223> sense-loop-sense Oligodesoxynukleotid für Luciferase
<400> 1
   gagctgtttc tgaggagcct tcaagagagg ctcctcagaa acagctc 47
<210> 2
   <211> 99
   <212> DNA
   <213> Homo sapiens
<220>
   <221> misc_feature
   <223> Promotor des Human Gene for H1 RNA
<400> 2
   atatttgcat gtcgctatgt gttctgggaa atcaccataa acgtgaaatg tctttggatt 60
   tgggaatctt ataagttctg tatgagagca cagataggg 99
<210> 3
   <211> 20
   <212> DNA
   <213> Artificial
<220>
   <223> synthetisches Oligodesoxynukleotid
<220>
   <221> misc_feature
   <223> Haarnadel-Oligo
<400> 3
   gggagtccag ttttctggac 20
<210> 4
   <211> 24
   <212> DNA
   <213> Artificial
<220>
   <223> synthetsiches Oligodesoxynukleotid
<220>
   <221> misc_feature
   <223> Haarnadel-Oligo
<400> 4
   tggaaagtcc agttttctgg actt 24
<210> 5
   <211> 20
   <212> DNA
   <213> Artificial
<220>
   <223> synthetisches Oligodesoxynukleotid
<220>
   <221> misc_feature
   <223> Haarnadel-Oligo
<400> 5
   aggggtccag ttttctggac 20

## Beschreibung

Die Erfindung betrifft ein Verfahren zur Herstellung von Vektoren, die geeignet sind, nach ihrer Transfektion in eukaryote Zellen, in diesen die Bildung definierter Proteine durch RNA -Interferenz gezielt zu inhibieren.

Eine kürzlich nachgewiesene Möglichkeit der Hemmung der Genexpression beruht auf der Erzeugung von doppelsträngigen RNA-Molekülen. Mit dieser Doppelstrang-RNA (dsRNA) lassen sich hochwirksam und schneller als mit jedem anderen Verfahren einzelne Gene gezielt ausschalten, ohne die Proteinbildung benachbarter Gene zu stören. Das zugrundeliegende Prinzip wird als RNA-Interferenz, kurz RNAi, bezeichnet, die dieses Phänomen verursachende dsRNA-Sequenz als siRNA (small interference RNA).

Die siRNA verhindert nicht das Ablesen des Gens, sondern schaltet einen zelleigenen Mechanismus an, der die vom Gen abgelesenen mRNA's abbaut und so die Bildung des entsprechenden Proteins unterbindet (post-transcriptional gene silencing).

Ausgelöst wird dieser gezielte mRNA-Abbau durch kurze siRNA-Moleküle mit 19-23 RNA-Basen Länge, die homolog zu der Target-mRNA sind, deren Umsetzung in ein Protein verhindert werden soll. Die siRNA-Moleküle setzen sich mit speziellen Endoribonukleasen zu einem zelleigenen RNA-Proteinkomplex mit der Bezeichnung "RISC" (RNA-induced silencing complex) zusammen. Bei dem Aufbau dieser Komplexe dissoziieren die beiden RNA-Stränge voneinander, wodurch sogenannte akti-aktivierte RISC entstehen, die jeweils einen Einzelstrang des siRNA-Moleküls enthalten. Aktivierte RISC, welche den antisense-Strang enthalten, der komplementär zu der Target-mRNA ist, binden an diese und die Endoribonuklease des RNA-Proteinkomplexes sorgt nun für den sequenzspezifischen mRNA-Abbau.

Die siRNA kann in der Zelle experimentell erzeugt werden oder durch Einschleusen von außen eingebracht werden. Zum Einen gelingt das über synthetisch hergestellte siRNA-Moleküle, die sowohl in-vitro und in-vivo verabreicht werden können.

Diese Methode hat jedoch technische Grenzen. Neben der generellen Instabilität der synthetischen siRNA im Medium als auch in der Zelle, ist die Inhibierung mittels synthetischer siRNA prinzipiell nur transient möglich und viele Zellen (z.B. neuronale Zellen) lassen sich nur sehr ineffizient transfizieren. Studien, die auf der Transfektion mit synthetischer siRNA beruhen, sind daher in der Regel sowohl zeitlich auf 1 - 5 Tage als auch Zelltyp-spezifisch eingeschränkt. Im Weiteren sind die hohen Produktionskosten und die lange Produktionsdauer nachteilig.

Zum Anderen kann siRNA durch Vektoren in der Zelle erzeugt werden. Dabei handelt es sich um virale oder plasmidbasierte Vektoren, die erst in der Zelle durch Expression zur Bildung der siRNA-Sequenzen führen. Die Vorteile gegenüber der Transfektion mit synthetischer siRNA liegen in der stabileren und ggf. regulierteren Transkription der entsprechenden siRNA-Sequenz.

So offenbart D1 beispielsweise ein Plasmid mit einer Transkriptionseinheit für RNA-Interferenz, bestehend aus einen durchgängig doppelsträngigen, zirkulären DNA-Strang als Vektor, wobei die Transkriptionskassette aus einem Promoter, der Gensequenz und einem Spacer besteht. Die Transkriptionseinheit eines Plasmids gemäß D1 besteht aus einem Polymerase-III H1-RNA Genpromoter, sowie einem genspezifischen Insert (19 Nukleotide), welches durch einen Spacer von seiner reversen komplementären Sequenz getrennt ist. Zusätzlich befinden sich hinter der komplementären Kopie der Gensequenz fünf Thymidine als Terminationssignal. Die gesamte Transkriptionseinheit ist, wie auch der Vektor, durchgängig aus DNA-Doppelstrang aufgebaut.

Jedoch zeigen die plasmidbasierten Vektoren neben einer niedrigen Transfektionseffizienz ebenfalls einen aufwendigen Produktionsprozeß. So ist es bspw. notwendig, stabile Klone zu selektieren. In diesem oftmals langwierigen Prozeß, der Wochen aber auch Monate dauern kann, kommt es häufig zum Auftreten zahlreicher potentieller Schwierigkeiten, die Klonierungsexperimenten innewohnen. Zur Überprüfung des Produkts sind Sequenzierungen notwendig, die ebenfalls arbeits- und kostenintensiv sind.

Des Weiteren enthalten die plasmidbasierten Vektoren Antibiotika-Resistenzgene, die zu ihrer Selektion notwendig sind. Aus diesem Grund sind derartige Vektoren nicht zur Anwendung in lebenden Organismen geeignet. Die mögliche Rekombination mit ubiquitär im Organismus vorkommenden Bakterien, birgt das Risiko eines zunehmenden Auftretens antibiotikaresistenter Bakterien in sich. Die Verbreitung von Antibiotikaresistenzen ist ein schwerwiegendes Problem und nicht vertretbar.

Da virale Vektoren zur effizienten und gezielten Transfektion fähig sind, bieten sie einen Vorteil gegenüber synthetischen siRNA-Molekülen als auch plasmidbasierten Vektoren.

Für die therapeutische Anwendung sind derartige virale Vektoren jedoch nur mit Vorbehalt einsetzbar. Auch hier stellt die Rekombination viraler Sequenzen mit natürlich vorkommenden Viren ein inhärentes Sicherheitsrisiko dar, da die Erzeugung neuer, pathogener Hybridviren befürchtet werden muß. Zudem ist auch ihre Herstellung aufwendig und kostenintensiv.

Eine weitere Möglichkeit Vektoren für siRNA herzustellen, zeigt die Firma Ambion in ihrer Internetpräsenz auf. Der dargestellte Prozess vermeidet die oben genannten Nachteile. Jedoch ist auch dieser Produktionsprozeß zeitaufwendig und durch eine Reihe an notwendigen Vervielfältigungsschritten der betreffenden Sequenzen mittels PCR (polymerase chain reaction) sehr fehlerbehaftet. Die Möglichkeit der Erzeugung sowohl unerwünschter als auch unbemerkter Mutationen, die durch den PCR-Prozeß noch potenziert werden, ist sehr groß. So sind auch hier Kontrollsequenzierungen, die den Herstellungsprozeß verlängern und zu erhöhten Kosten beitragen, nötig.

Ausgehend von diesem Stand der Technik ist es Aufgabe der Erfindung ein geeignetes Verfahren zur in-vitro oder in-vivo Synthese einer definierten siRNA-Sequenz zur Verfügung zu stellen.

Diese Aufgabe wir durch die kennzeichnenden Merkmale der Ansprüche 1 und 9 gelöst.

Im Sinne der vorliegenden Erfindung soll unter siRNA-Sequenz die RNA-Sequenz verstanden werden, die von dem erfindungsgemäß hergestellten DNA-Konstrukt abgelesen wird. Es handelt sich dabei somit um einen singulären RNA-Einzelstrang, der partiell selbstkomplementär ist.

Im Sinne der vorliegenden Erfindung wird die Bezeichnung siRNA-Molekül für eine siRNA verwendet, welche aufgrund der Rückfaltung und Basenpaarung einer selbstkomplementären siRNA-Sequenz entsteht. Es handelt sich daher bei einem siRNA-Molekül um ein doppelsträngiges RNA-Molekül, bei dem die miteinander paarenden Stränge an einer Seite durch einen nicht komplementären Einzelstrang verbunden sind.

Erfindungsgemäß ist ein Verfahren vorgesehen, welches durch die folgenden Schritte gekennzeichnet ist
a) Partielles Annealing von einem partiell selbstkomplementären Oligodesoxinukleotid zur Bildung eines DNA-Doppelstranges, welcher eine 19 - 23 lange, singuläre Kopie einer Gensequenz einmal in 5' - 3'-Richtung und einmal in 3' - 5'-Richtung enthält, wobei zwischen der 5' - 3'- und 3' - 5'-Orientierung der singulären Kopie der Gensequenz jeweils eine nicht selbstkomplementäre 8 - 12 Basen lange Sequenzfolge von zwei Einzelsträngen angeordnet ist, welche so gewählt sind, dass gegenüberliegende Basen in keinem Fall komplementär zueinander sind und die flankierenden Doppelstrangbereiche so durch zwei DNA-Einzelstränge miteinander verbunden sind, wobei der DNA-Doppelstrang an den Enden kurze überstehende Enden einzelsträngiger DNA aufweist und Mischung des DNA-Doppelstranges mit
   haarnadelförmigen Oligodesoxynukleotiden, welche an den Enden kurze überstehende Enden einzelsträngiger DANN aufweisen
   und
   einem Promotor mit kurzen überstehenden Enden einzelsträngiger DNA, wobei das einzelsträngige 5'-Ende des Promotors mit einem der haarnadelförmigen Oligodesoxynukleotide paaren kann und das einzelsträngige 3'-Ende des Promoters komplementär zu dem einzelsträngigen 5'-Ende des DNA-Doppelstranges ist
   und
   einem Terminationssignal für RNA-Polymerasen mit kurzen überstehenden Enden einzelsträngiger DNA, wobei der 5'-Überhang des Terminationssignals mit dem 3'-Ende des DNA-Doppelstranges spezifisch paaren kann und der 3'-Überhang des Terminationssignals mit einem haarnadelförmigen Oligodesoxynukleotid spezifisch paaren können, und
b) anschließender Ligation der DNA-Fragmente, sowie
c) abschließender Aufreinigung der hergestellten Vektoren.

In einer bevorzugten Ausführungsform ist ein Verfahren vorgesehen, bei dem der Promotor Teil eines bakteriell amplifizierbaren Plasmides ist, welches vor der Mischung der Komponenten im ersten Verfahrensschritt 1 a) mit einer Restriktionsendonuklease geschnitten wird, welche eine den Promotor auf dem Plasmid flankierende Schnittstelle erkennt, die nicht auf dem herzustellenden Molekül vorhanden ist.

Ferner ist es erfindungsgemäß vorgesehen, dass im Falle der Verwendung eines Promotors als Teil eines bakteriell amplifizierbaren Plasmides der Ligationsschritt in Anwesenheit der Restriktionsendonuklease erfolgt, mit welcher der Promotor aus dem Plasmid ausgeschnitten wurde.

In einer Ausführungsform wird vor dem abschließenden Aufreinigungsschritt ein Verdau der Reaktionsmischung mittels einer ausschließlich für 3'- oder 5'-DNA-Enden spezifischen Exonuklease durchgeführt.

Bei dem erfindungsgemäßen Verfahren kann der DNA-Doppelstrang, welcher zu Beginn der Mischung zugegeben wird, aus dem Annealing von einem partiell selbstkomplementären Oligodesoxynukleotiden resultieren. Das Annealing kann auch erst in der Reaktionsmischung erfolgen, so dass zu Beginn des erfindungsgemäßen Verfahrens lediglich einzelsträngige komplementäre Oligodesoxynukleotide zugegeben werden.

Die Sequenz der Oligodesoxynukleotide ist in einer bevorzugten Ausführungsform so gewählt, dass die daraus resultierenden Haarnadeln in ihrem doppelsträngigen Bereich die Erkennungssequenz für eine Restriktionsendonuklease aufweisen.

Die abschließende Aufreinigung der mittels des erfindungsgemäßen Verfahren hergestellten Vektoren erfolgt bevorzugt entweder durch Chromatographie oder Gelelektrophorese.

Wird der Promotor als Teil eines bakteriell amplifizierbaren Plasmides in das erfindungsgemäße Herstellungsverfahren eingesetzt, so handelt es sich bei der Restriktionsendonuklease, mit welcher der Promotor aus dem Plasmid ausgeschnitten werden kann, um ein Enzym der Gruppe der Klasse-II-Restriktionsendonukleasen, vorzugsweise aus der Gruppe BbsI, Bbvl, Bbvll, Bpil; Bpll, Bsal, BsmAI, BsmBI, BsmFI, BspMI, Eam1104I, EarI, Eco31I, Esp3I, Fokl, Hgal, SfaNI oder deren Isoschizomere.

Offenbart ist ein Kit zur Durchführung des erfindungsgemäßen Verfahrens enthaltend wenigstens einen Promotor, haarnadelförmige Oligodesoxynukleotide und Enzyme. Bei den Enzymen handelt es sich um Ligasen, Restriktionsendonukleasen, Restriktionsexonukleasen, Kinasen und Polymerasen oder ausgewählte Kombinationen davon, in Form eines Enzymmixes. Zusätzlich kann der Kit je nach Ausführungsform noch Mittel zur Durchführung der enzymatischen Reaktionen enthalten sowie Mittel zur Aufreinigung der hergestellten Vektoren. Der Promotor kann im Kit als Teil eines Plasmids enthalten sein, aus dem dieser unter Verwendung einer geeigneten Restirktionsendonuklease herausgeschnitten werden kann.

Des weiteren ist ein Vektor Gegenstand der vorliegenden Erfindung, der nach dem erfindnungsgemäßen Verfahren hergestellt wurde und der durch haarnadelförmige Oligodesoxynukleotide abgeschlossen ist, zwischen denen ein Promoter am 5'-Ende und ein Terminationssignal am 3'-Ende eines DNA-Doppelstranges angeordnet ist, wobei der DNA-Doppelstrang eine 19 - 23 Basen lange, singuläre Kopie einer Gensequenz einmal in 5' - 3'-Richtung und einmal in 3' - 5'-Richtung enthält, wobei zwischen der 5' - 3'- und 3' - 5'-Orientierung der singulären Kopie der Gensequenz jeweils eine nicht selbstkomplementäre 8 - 12 Basen lange Sequenzfolge von zwei Einzelsträngen angeordnet ist, welche so gewählt sind, dass gegenüberliegende Basen in keinem Fall komplementär zueinander sind und die flankierenden Doppelstrangbereiche so durch zwei DNA-Einzelstränge miteinander verbunden sind. Diese Expressionskassetten werden auch als Minimalistic siRNA Expression Cassettes (MISECs) bezeichnet.

Von dem bekannten Stand der Technik unterscheidet sich die vorliegende Erfindung dadurch, dass ein schnelles Verfahren zur Verfügung gestellt wird, mit dessen Hilfe ein Vektor hergestellt wird, der frei von Plasmid - oder viralen Bestandteilen ist und zur Expression von siRNA-Sequenzen führt. Das Verfahren zur Herstellung derartiger Vektoren beinhaltet keine PCR-Schritte, ist eine Dreischrittprozedur in einem Reaktionsgefäß und in wenigen Stunden durchführbar. Damit steht ein Verfahren zur Verfügung, mit dessen Hilfe man in kürzester Zeit sehr einfach verschiedenste siRNA-Sequenzen auf deren Funktionalität prüfen kann. Screeningverfahren nach geeigneten siRNA-Sequenzen können unter Nutzung der schnellen und unkomplizierten Herstellung der Vektoren mit Hilfe des Kits kosten-u. zeitsparend durchgeführt werden. Ein weiterer Vorteil der so erzeugten Vektoren ist ihre Kleinheit, die unter anderem die Transfektion erleichtert.

Die siRNA-Sequenzen sind einzelsträngig und bestehen aus einem sense und einem antisense Strang, der jeweils 19-23 Nukleotide umfaßt. Sense -u. antisense Strang sind durch eine kurze Spacerregion getrennt, die die spätere Faltung der Stränge zu einem doppelsträngigen siRNA-Molekül erlaubt. Diese siRNA paart mit einer Target-mRNA und führt wie beschrieben zu deren Abbau durch Nukleasen.

Der mit dem Herstellungsverfahren erzeugte Vektor besteht lediglich aus einer geeigneten Promotorsequenz, der zu exprimierenden siRNA-Sequenz und einer kurzen Terminationssequenz und trägt somit keine unerwünschten Sequenzen viraler oder plasmidischer Herkunft. Zum Schutz vor Abbau durch Exonukleasen werden die Enden mit je einer Schlaufe aus einzelsträngigen Oligodinukleotiden (ODN) kovalent verknüpft, so daß ein durchgehend kovalent geschlossenes Molekül entsteht.

Als Promotor für die Transkriptionskontrolle eignet sich prinzipiell jede eukaryote Promotorsequenz wie z. B. der CMV-Promotor des Cytomegalovirus. Bevorzugt werden Typ III Polymerase Promotoren eingesetzt, wie beispielsweise der H1 Promotor, der 7SK Promotor sowie der humane und murine U6 Promotor. Die Promotorsequenz kann auf einem geeigneten Plasmidvektor vorliegen, aus dem sie durch Restriktionsendonukleasen zu Herstellungsbeginn ausgeschnitten werden muß, die Promotorsequenz kann jedoch auch als schon isolierte oder synthetisch hergestellte Sequenz dem Verfahren zugesetzt werden.

Als Terminationssequenz kommt jegliche bekannte DNA-Sequenz in Frage, die zum Abbruch der Expression durch RNA-Polymerasen führt. Die Terminationssequenz muss nicht extra dem erfindungsgemäßen Verfahren zugesetzt werden, sondern kann auch Teil des doppelsträngigen Bereichs eines haarnadelförmigen Oligodesoxynukleotids sein oder des 3'-Endes des partiellen DNA-Doppelstranges, welcher zentral zwei Einzelstränge aufweist.

Die zu exprimierende siRNA-Sequenz ist die zur Target-mRNA komplementäre Sequenz, die als PCR-Produkt in das erfindungsgemäße Herstellungsverfahren eingesetzt wird. Ebenso kann die siRNA-Sequenz synthetisch durch Oligodinukleotidsynthese hergestellt werden. Dabei kann es sich um kurze ODN-Fragmente handeln, die in einem ersten Schritt annealt und ligiert werden müssen, es kann jedoch auch die gesamte siRNA-Sequenz durch ODN-Synthese erzeugt werden. Das ODN-Fragment wird durch die PNKinase phosphoryliert.

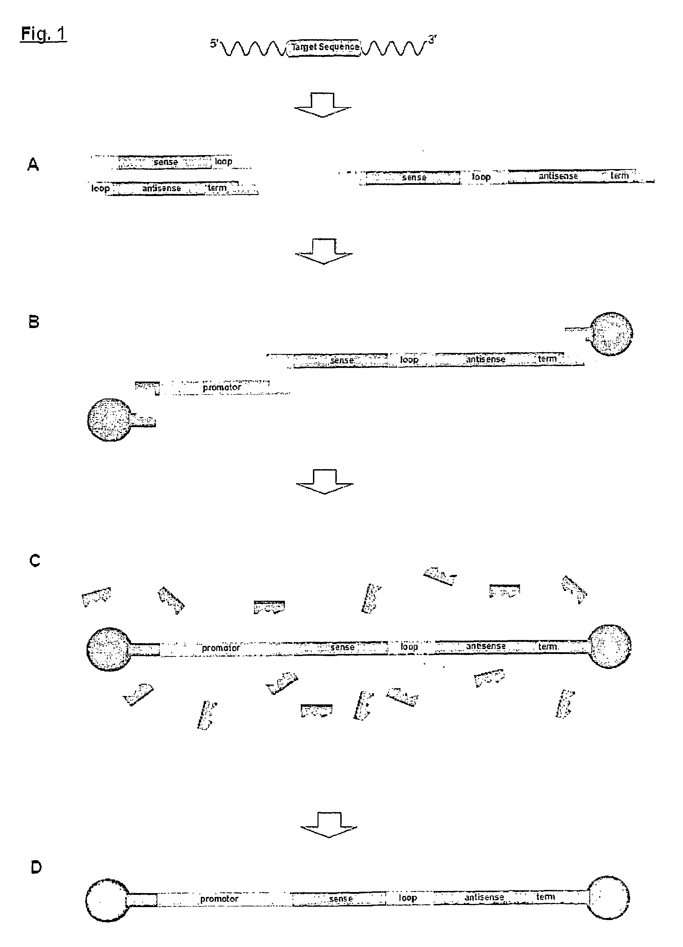

Der Herstellungsprozeß des erfindungsgemäßen Verfahrens ergibt sich wie folgt und ist in Fig. 1 zur Übersicht schematisch dargestellt.

Das Plasmid, das die Promotorsequenz trägt, wird mit dem Restriktionsenzym BspTNI über Nacht bei 37°C vollständig verdaut und somit das Promotorfragment bereitgestellt. Nach Zugabe der betreffenden siRNA-Sequenz sowie der 5' phosphorylierten haarnadelförmigen Oligodesoxynukleotide im zweifachen Überschuß, werden mit Hilfe des Enzymes T4-DNA Ligase in Anwesenheit des Restriktionsenzymes BspTNI die einzelnen Fragmente ligiert. Das resultierende Gemisch an Nukleinsäuren wird mit dem Enzym T7-DNA-Polymerase behandelt. Das Endprodukt, der siRNA exprimierende Vektor, wird durch Säulenchromatographie aufgereinigt und steht zur Transfektion bereit.

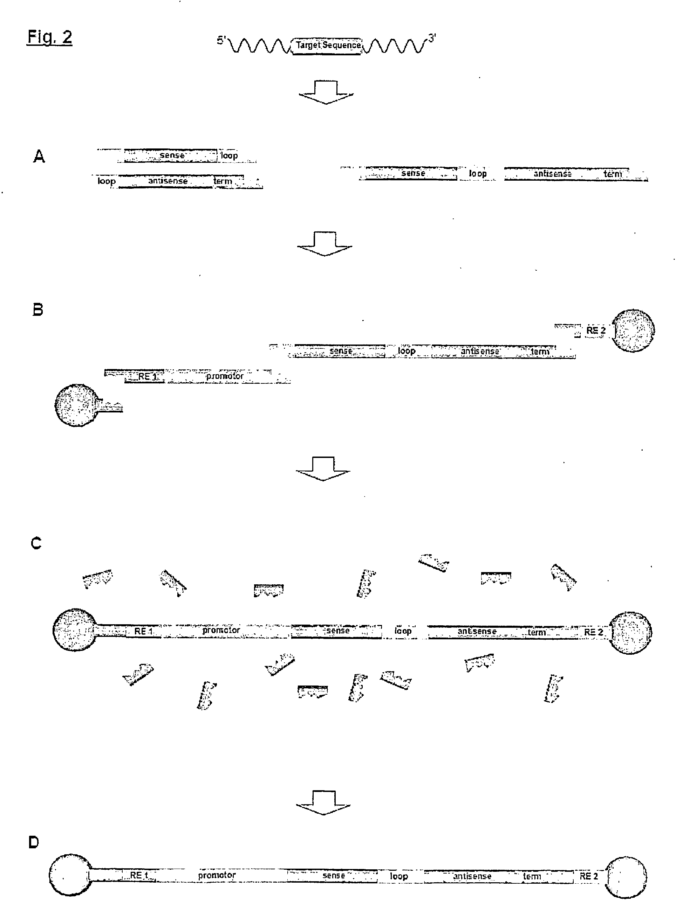

In einer Ausführungsform ist vorgesehen, dass der siRNA-exprimierende Vektor zwei Restriktionsschnittstellen enthält, die das spätere Abtrennen der Haarnadeln erlauben. Das ist von Vorteil, da der Vektor damit weiteren Prozessen zugänglich ist. Sei es das Klonieren der Sequenz in einen beliebigen Expressionsvektor, bspw. ein Plasmid, sei es die Vervielfältigung der Sequenz durch PCR oder ähnliches. Diese Ausführungsform ist in Fig. 2 gezeigt.

Ein in-vitro Versuch zur Überprüfung der Funktionalität der erfindungsgemäßen siRNA-Vektoren wurde durchgeführt. Dazu wurden Hamsterzellen mit verschiedenen siRNA-Vektoren transfiziert, die die Expression der Luciferase unterdrücken sollten. Als siRNA-Vektoren wurden Plasmid und erfindungsgemäßer Vektor eingesetzt. Beide Vektoren erreichten eine ca. 90% Hemmung der Luciferaseexpression. Bei vergleichbarer Wirksamkeit und Transfektionseffizienz gelingt es vorteilhafterweise durch das erfindungsgemäße Verfahren, einen Vektor herzustellen, der die beschriebenen Nachteile der plasmidbasierten Vektoren vermeidet und wesentlich zeit-u. kostensparender hergestellt werden kann.

Die Bedeutung der Erfindung ergibt sich somit in der Bereitstellung eines Verfahrens zur Herstellung geeigneter Vektoren, die zu Screeningverfahren eingesetzt werden können und so zur schnellen funktionellen Überprüfung potentieller siRNA-Sequenzen dienen. Der Kit stellt weiterhin ein potentielles Werkzeug für die Gentherapie, im Sinne der Abschaltung krankhafter Gene, zur Verfügung.

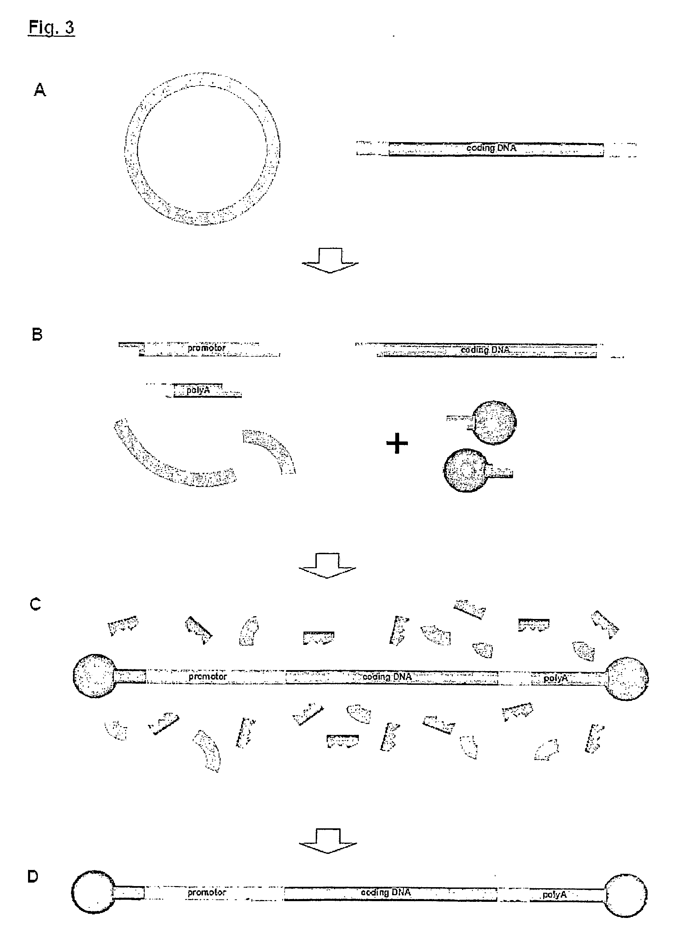

Mit dem Herstellungsverfahren können jedoch auch auf einfachem Weg DNA-Expressionsvektoren hergestellt werden. Dazu wird die siRNA-Sequenz durch eine für ein Gen kodierende DNA-Sequenz ersetzt. Durch Restriktionsverdau werden an den Enden der DNA-Sequenz einzigartige Überhänge geschaffen, die es erlauben, die Fragmente (Promotor, polyA-site und haarnadelförmige ODN) in der richtigen Anordnung zu ligieren. Fig. 3 zeigt schematisch diesen Herstellungsweg. Ein Kit, der die Herstellung der DNA-Expressionsvektoren erlaubt, ist ebenfalls vorgesehen. Bestandteile des Kits sind: geeignetes Plasmid mit Promotor-und polyA-site-Sequenz, kodierende DNA-Sequenz, haarnadelförmige ODN, ATP, Ligase, Restriktionsenzym, T7-Polymerase sowie Chromatographiesäulenmaterial zur Aufreinigung des Produktes.

Weitere vorteilhafte Maßnahmen sind in den übrigen Unteransprüchen beschrieben; die Erfindung wird anhand von Ausführungsbeispielen und den nachfolgenden Figuren näher beschrieben; es zeigt:
Fig. 1: zeigt den Herstellungsweg der siRNA-Vektoren
   A: in das Verfahren einzusetzende siRNA-Sequenz, die homolog zur Target-mRNA ist. Die Sequenz besteht aus dem sense-antisense-loop- Bereich und einer Terminationssequenz. Die siRNA-Sequenz kann aus einzelnen ODN-Fragmenten, die mit Hilfe des Enzymmix annealt, ligiert u. ggf. phosphoryliert werden müssen, bestehen, sie kann auch schon als vollständiges ODN-Fragment vorliegen.
   B: zeigt die Bestandteile, die durch das Enzym Ligase an das ODN-Fragment ligiert werden. Dabei handelt es sich um die Promotorsequenz mit entsprechenden komplementären Überhängen und um haarnadelförmige Oligodinukleotide, deren wiederum komplementären und einzigartigen Überhänge von jeweils 4 Basen dazu führen, das ein kovalent geschlossener linearer Vektor entsteht, der aus Promotor, Sense-, Loop-, Antisense- und Terminationssequenz besteht und an den Enden haarnadelförmig geschlossen ist.
   C: in einem letzten Schritt werden unligierte Bestandteile durch T7-DNA Polymerase Verdau abgebaut. Das zurückbleibende Produkt wird säulenchromatographisch aufgereinigt.
   D: zur Transfektion bereites Endprodukt
Fig. 2: zeigt den Herstellungsweg der siRNA-Vektoren mit zusätzlichen Restriktionsschnittstellen
   **A:** in das Verfahren einzusetzende siRNA-Sequenz, die homolog zur Target-mRNA ist. Die Sequenz besteht aus dem sense-antisense-loop- Bereich und einer Terminationssequenz. Die siRNA-Sequenz kann aus einzelnen ODN-Fragmenten, die mit Hilfe des Enzymmix annealt, ligiert u. ggf. phosphoryliert werden müssen, bestehen, sie kann auch schon als vollständiges ODN-Fragment vorliegen.
   **B:** zeigt die Bestandteile, die durch das Enzym Ligase an das ODN-Fragment ligiert werden. Dabei handelt es sich um die Promotorsequenz mit entsprechenden komplementären Überhängen, die mit einer zusätzlichen Restriktionsschnittstelle am 5' Ende versehen wurde, und um haarnadelförmige Oligodinukleotide, wobei ein haarnadelförmiges ODN ebenfalls eine zusätzliche Restriktionsschnittstelle trägt, deren wiederum komplementären und einzigartigen Überhänge von jeweils 4 Basen dazu führen, das ein kovalent geschlossener linearer Vektor entsteht, der aus Promotor, Sense-, Loop- , Antisense- und Terminationssequenz besteht und an den Enden haarnadelförmig geschlossen ist.
   **C:** in einem letzten Schritt werden unligierte Bestandteile durch T7-DNA Polymerase Verdau abgebaut. Das zurückbleibende Produkt wird säulenchromatographisch aufgereinigt.
   **D:** zur Transfektion bereites Endprodukt mit zwei Restriktionsschnittstellen
**Fig. 3****:** zeigt den Herstellungsweg DNA-kodierender Vektoren
   **A:** Als Ausgangsmaterial dient ein Plasmid sowie eine kodierende DNA-Sequenz. Das Plasmid trägt Restriktionsschnittstellen, die das Ausschneiden der Promotor-und der polyA-site-Sequenz ermöglichen.
   **B:** durch Restriktionsverdau entstehen die Fragmente: Promotor und polyA-site, die jeweils einzigartige komplementäre Überhänge aufweisen sowie Rest-Plasmidfragmente. Nach Zugabe der kodierenden DNA-Sequenz, haarnadelförmiger ODN und in Anwesenheit des Enzyms Ligase werden die Fragmente ligiert.
   **C:** unligierte Bestandteile werden durch T7-DNA Polymerase Verdau abgebaut. Der kovalent geschlossene Vektor, bestehend aus Promotor- kodierender-und polyA-site-Sequenz wird säulenchromatographisch aufgereinigt.
   **D:** zur Transfektion einsetzbarer DNA-exprimierender Vektor.

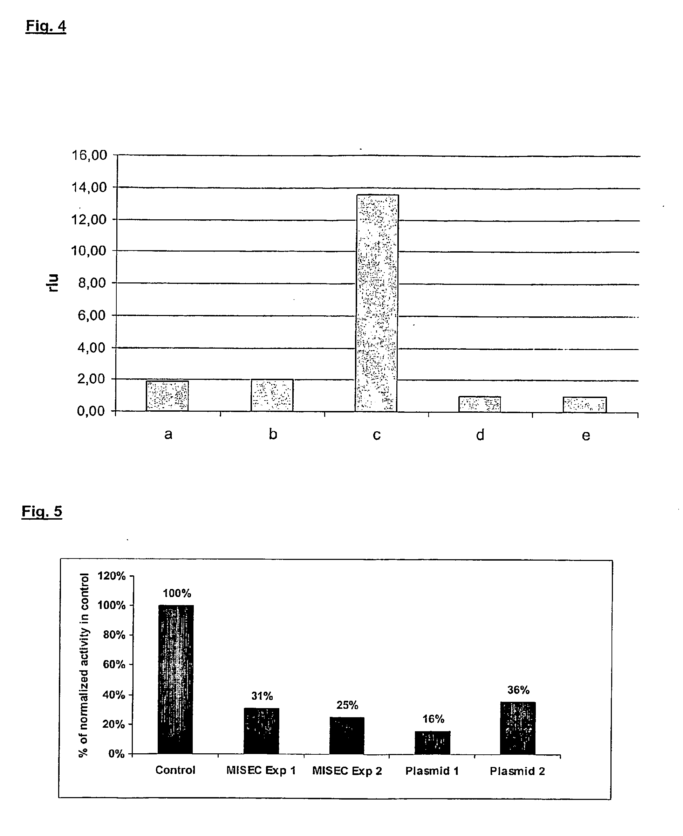

**Fig. 4****:** zeigt die Hemmung der Luciferaseexpression in-vitro durch siRNA
   Die Expression wurde anhand von relativen Lichteinheiten (rlu) nach Transfektion von CHOK1-Zellen bestimmt. Eingesetzt wurden: mit erfindungsgemäßem Verfahren hergestellter siRNA-Vektor (a), siRNA-Sequenz tragendes Plasmid (b), Positivkontrolle zur Kontrolle der Luciferaseexpression (c), unbehandelte Zellen (d) und mit Leervektor transfizierte Zellen (e). Die Werte stellen Mittelwerte dar, die aus Mehrfachbestimmungen errechnet wurden. Bei den Negativ-und der Positivkontrollen wurde die Unterdrückung der Luciferaseexpression erwartungsgemäß nicht beobachtet. Dagegen zeigten die mit siRNA-behandelten Zellen eine signifikant geringere Luciferaseexpression. Die Luciferaseexpression ist bis zu 90% herabgesetzt im Vergleich zur Positivkontrolle.
**Fig. 5** zeigt die Ergebnisse eines Experiments, bei dem der erfindungsgemäße siRNA Expressionsvektor mit Plasmiden verglichen wird, die die identischen Expressionskassetten enthalten.
   CHO-K1 Zellen wurden mit 0.5 ng Plasmid, welches für Renilla Luziferase kodiert und 4.5 ng Plasmid, welches für die Firefly Luziferase kodiert und 195 ng des korrespondierenden siRNA Expressionskonstruktes, welches gegen die Expression der Firefly Luziferase gerichtet war, kotransfiziert. 24 Stunden nach der Transfektion wurden die Zellen lysiert und in einem Luminometer die Luziferase-Aktivität bestimmt. Die Aktivität der Firefly Luziferase wurde gegen die Aktivität der Renilla Luziferase abgeglichen und mit der Aktivität der Kontrolle verglichen (unspezifische siRNA). Die dargestellten Ergebnisse zeigen die Mittelwerte von drei unabhängigen Versuchen.
   Die Unterdrückung der Genexpression durch die MISECs, welche mit einem "siRNA Expression Vector Kit" unter Verwendung des erfindungsgemäßen Verfahrens hergestellt wurden, ist vergleichbar mit den Effekten der Plasmid-Transfektionen und die Unterdrückung der Genexpression liegt bei beiden Transfektionen im Bereich zwischen 70 - 75%.

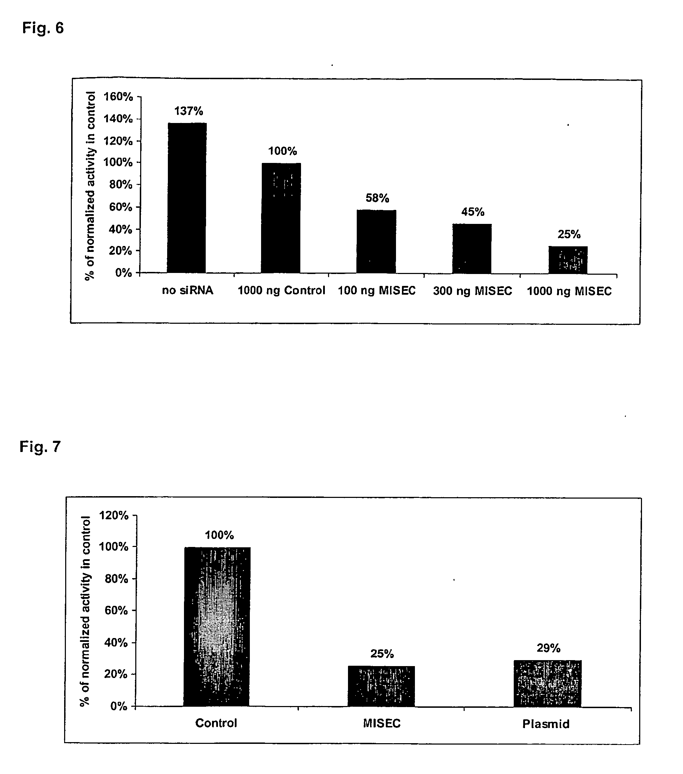

**Fig. 6****:** zeigt die Dosisabhängigkeit der Genrepression nach der Transfektion von MISECs, welche mit einem "siRNA Expression Vector Kit" hergestellt wurden, wobei die Haamadel-siRNA gegen das Firefly Luziferasegen gerichtet war, im Vergleich zu nicht spezifischen siRNA Sequenzen.
   CHO-K1 wurden mit 500 ng Plasmid kodierend für Renilla Luziferase, 100 ng Plasmid kodierend für Firefly Luziferase und siRNA Expressionskonstrukten gegen das Firefly Luziferasegen mit den angegeben Mengen kotransfiziert. Nach 24 Stunden wurden die Zellen lysiert und in einem Luminometer die Aktivität der Luziferasen bestimmt. Die Aktivität der Firefly Luziferase wurde gegen die Aktivität der Renilla Luziferase abgeglichen und mit der Aktivität der Kontrolle verglichen (unspezifische siRNA). Die dargestellten Ergebnisse zeigen die Mittelwerte von zwei unabhängigen Versuchen.
   Mit der unspezifischen siRNA verglichen, zeigt die Transfektion der gleichen Menge (1000 ng) der mit einem "siRNA Expression Vector Kit" unter Verwendung des erfindungsgemäßen Verfahrens hergestellten MISECs eine signifikante Abnahme der Luziferase-Aktivität.
**Fig. 7****:** In einem weiteren Experiment, welches in der Arbeitsgruppe von Dr. Christiane Kleuss am Institut für Pharmakologie der Freien Universität Berlin durchgeführt wurde, wurde die Effektivität der Genrepression durch MISECs, welche mit einem "siRNA Expression Vector Kit" unter Verwendung des erfindungsgemäßen Verfahrens hergestellt wurden, mit einem Plasmid verglichen, welches die Gleiche Expressionskassette enthält.
   CHO-K1 wurden mit 12 ng Plasmid kodierend für Renilla Luziferase, 6 ng Plasmid kodierend für Firefly Luziferase und 182 ng eines korrespondierenden siRNA Expressionskonstruktesgegen das Firefly Luziferasegen kotransfiziert. CHO-K1 wurden mit 500 ng Plasmid kodierend für Renilla Luziferase, 100 ng Plasmid kodierend für Firefly Luziferase und siRNA Expressionskonstrukten gegen das Firefly Luziferasegen mit den angegeben Mengen kotransfiziert.
   Nach 24 Stunden wurden die Zellen lysiert und in einem Luminometer die Aktivität der Luziferasen bestimmt. Die Aktivität der Firefly Luziferase wurde gegen die Aktivität der Renilla Luziferase abgeglichen und mit der Aktivität der Kontrolle verglichen (unspezifische siRNA). Die dargestellten Ergebnisse zeigen die Mittelwerte von drei unabhängigen Versuchen.
   Auch in diesem Experiment zeigen die siRNA Expressionsvektoren, welche nach dem erfindungsgemäßen Verfahren hergestellt wurden, die gleiche Effektivität wie das Plasmid mit der identischen Expressionskassette, welche jeweils bei ca. 75% Reduktion der Luziferaseaktivität gegenüber dem unspezifischen Kontrollplasmid liegt. Die Inhibition der erfindungsgemäße hergestellten Konstrukte ist vollkommen ausreichend, um in einem Screeningverfahren in kurzer Zeit effektiv erfolgreiche Zielsequenzen, welche zur Genrepression geeignet sind, zu identifizieren.

### Beispiel 1: Herstellung von siRNA-Vektoren zur Unterdrückung der Luciferase Expression

Die für die siRNA der Luciferase (siRNALuc) kodierenden Vektoren wurden wie folgt erhalten:
Die zwei ODN-Fragmente für siRNALuc wurden bei 90°C für 3min erhitzt und durch langsames Abkühlen annealt. Dadurch wurde folgende für Luciferase kodierende Sequenz erhalten:

Dabei bilden die 19 ersten Basen den sense Strang, die folgenden 9 Basen den loop Bereich und die restlichen 19 Basen den antisense Strang.

Die Phosphorylierung durch PNKinase erfolgte im Anschluß. Zum Erhalt von 10 Mikrogramm Endprodukt wurden 3,9 Mikrogramm siRNALuc eingesetzt. Nach Zugabe von 5,2 Mikrogramm H1-Promotor (Seq. ID 2) sowie der 5'-phosphorylierten haarnadelförmigen Oligodesoxynukleotide (Seq. ID 3 und 4):

Seq. ID 3: 5'-PH-GGG AGT CCA GTT TTC TGG AC-3' (1,2 Mikrogramm) und
Seq. ID 4: 5' PH-TGG AAA GTC CAG TTT TCT GGA CTT-3' (1,4 Mikrogramm)
wurden mit Hilfe des Enzymes T4-DNA Ligase in Anwesenheit des Restriktionsenzymes BspTNI die einzelnen Fragmente ligiert. Das resultierende Gemisch an Nukleinsäuren wurde mit dem Enzym T7-DNA-Polymerase behandelt. Das Endprodukt, der siRNALuc exprimierende Vektor, wurde durch Säulenchromatographie aufgereinigt und stand zur Transfektion bereit.

### Beispiel 2: Unterdrückung der Luciferase Expression in-vitro

Hamsterzellen der CHOK1 Zelllinie wurde in Platten mit 24 Löchern ausgesät, wobei pro Loch 8x10⁴ Zellen in 500 Mikroliter Medium ausgesät wurden. Nach der Inkubation über 24h erfolgte die Transfektion mit verschiedenen siRNALuc exprimierenden Konstrukten. Als Transfektionsreagenz diente FuGene6. Als Referenzvektor zur Bestimmung der Firefly- Luciferaseaktivität wurde in jeden Ansatz zusätzlich zu einem Firefly-Luciferase kodierenden Plasmid ein für die Renilla-Luciferase kodierendes Plasmid eingesetzt. Dadurch kann mit Hilfe eines Dual-Assays die Firefly-Luciferaseexpression im Verhältnis zur Marker-Renillaluciferaseexpression bestimmt werden. Als Negativkontrollen dienten untransfizierte Zellen und mit einem Leervektor transfizierte Zellen. Nach Übernacht-Inkubation wurden die Zellen lysiert und in je 15 Mikroliter Passiv-Lysispuffer aufgenommen. Der Nachweis der Expression erfolgte mittels eines Dual-Luciferase Reporter Assays im Luminumeter. Das Ergebnis ist in Figur 4 dargestellt

### Beispiel 3: Herstellung von für DNA-kodierende Vektoren mit dem erfindungsgemäßen Verfahren

Die DNA-Vektor Herstellung erfolgt im kombinierten Restriktions- / Ligationsansatz. Das eingesetzte Plasmid pMCV2.8 trägt vier BspTNI- bzw. Eco31I-Schnittstellen und stellt nach dem Verdau Promoter und polyA-site zur Verfügung.

Das Plasmid und das kodierende DNA-Fragment werden mit dem Restriktionsenzym BspTNI verdaut und mit den haarnadelförmigen ODN ligiert. Sequenzen der haarnadelförmigen ODN:
Seq.ID 3: 5' -PH-GGG AGT CCA GTT TTC TGG AC-3' und
Seq.ID 5:5' PH- AGG GGT CCA GTT TTC TGG AC-3'

Der Restriktions- / Ligationsansatz enthält somit: Plasmid, kodierende DNA- Sequenz, haarnadelförmige ODN, Reaktionspuffer, ATP, BspTNI sowie T4-DNA-Ligase. Die Inkubation erfolgt über 4 h bei 37°C. Der Prozeß wird durch Hitzeinaktivierung für 15' bei 70°C gestoppt.

Der Abbau des Restvektors sowie aller nicht ligierten Fragment erfolgt mittels T7-DNA-Polymerase Verdau. Der kodierende Vektor wird chromatographisch aufgereinigt und steht zur Transfektion bereit.

## Patentansprüche

1. Herstellungsverfahren für Vektoren, die nach ihrer Transfektion in eukaryote Zellen die Bildung definierter Proteine gezielt durch RNA-Interferenz inhibieren, **gekennzeichnet durch** die folgenden Verfahrensschritte
a) Partielles Annealing von einem partiell selbstkomplementären Oligodesoxynukleotid zur Bildung eines DNA-Doppelstranges, welcher eine 19 - 23 Basen lange, singulare Kopie einer Gensequenz einmal in 5' - 3'-Richtung und einmal in 3' - 5'-Richtung enthält, wobei zwischen der 5' - 3'- und 3' - 5'-Orientierung der singulären Kopie der Gensequenz jeweils eine nicht selbstkomplementäre 8 - 12 Basen lange Sequenzfolge von zwei Einzelsträngen angeordnet ist, welche so gewählt sind, dass gegenüberliegende Basen in keinem Fall komplementär zueinander sind und die flankierenden Doppelstrangbereiche so **durch** zwei DNA-Einzelstränge miteinander verbunden sind, wobei der DNA-Doppelstrang an den Enden kurze überstehende Enden einzelsträngiger DNA aufweist,
und Mischung des DNA-Doppelstranges
mit
haarnadelförmigen Oligodesoxynukleotiden, welche an den Enden kurze überstehende Enden einzelsträngiger DNA aufweisen
und
einem Promotor mit kurzen überstehenden Enden einzelsträngiger DNA, wobei das einzelsträng-ige 5'-Ende des Promotors mit einem der haarnadelförmigen Oligodesoxynukleotide paaren kann und das ein-zelsträngige 3'-Ende des Promotors komplementär zu dem einzelsträngigen 5'-Ende des DNA-Doppelstranges ist
und
einem Terminationssignal für RNA-Polymerasen mit kurzen überstehenden Enden einzelsträngiger DNA, wobei der 5'-Überhang des Terminationssignals mit dem 3'-Ende des DNA-Doppelstranges spezifisch paaren kann und der 3'-Überhang des Terminationssignals mit einem haarnadelförmigen Oligodesoxynukleotids spezifisch paaren kann,und
b) anschließender Ligation der DNA-Fragmente, sowie
c) abschließender Aufreinigung der hergestellten Vektoren.

2. Verfahren nach Anspruch 1, wobei der Promotor Teil eines bakteriell amplifizierbaren Plasmides ist, welches vor der Mischung der Komponenten in Verfahrensschritt 1 a) mit einer Restriktionsendonuklease geschnitten wird, welche eine den Promotor auf dem Plasmid flankierende Schnittstelle erkennt, die auf dem herzustellenden Molekül nicht vorhanden ist.

3. Verfahren nach Anspruch 2, wobei der Ligationsschritt nach Verfahrensschritt 1 b) in Anwesenheit der Restriktionsendonuklease erfolgt, mit welcher der Promotor aus dem Plasmid gespalten wurde.

4. Verfahren nach Anspruch 2 oder 3, wobei vor dem abschließenden Aufreinigungsschritt nach Verfahrensschritt 1 c) ein Verdau der Reaktionsmischung mittels einer ausschließlich für 3'- oder 5'-DNA-Enden spezifischen Exonuklease erfolgt.

5. Verfahren nach wenigstens einem der Ansprüche 2 bis 4, wobei es sich bei der Restriktionsendonuklease um ein Enzym der Gruppe der Klasse-II-Restriktionsendonukleasen, vorzugsweise ein Enzym aus der Gruppe
BbsI, BbvI, BbvII, BpiI; BplI, BsaI, BsmAI, BsmBI, BsmFI, BspMI, Eam1104I, EarI, Eco31I, Esp3I, FokI, HgaI, SfaNI oder deren Isoschizomere handelt.

6. Verfahren nach wenigstens einem der vorhergehenden Ansprüche, wobei es sich bei dem zugegebenen Promotor um den Promotor des humanen Genes für H1 RNA (SeqID.2) handelt.

7. Verfahren nach wenigstens einem der vorhergehenden Ansprüche, wobei die haarnadelförmigen Oligodesoxynukleotide in ihrem doppelsträngigen Bereich die Erkennungssequenz für eine Restriktionsendonuklease aufweisen.

8. Verfahren nach wenigstens einem der vorhergehenden Ansprüche, wobei die Aufreinigung in Verfahrensschritt 1 c) durch Chromatographie und/oder mittels Gelelektrophorese erfolgt.

9. Vektor, hergestellt nach wenigstens einem der Ansprüche 1 bis 8, der nach der Transfektion in eukaryote Zellen die Bildung definierter Proteine gezielt durch RNA-Interferenz inhibiert, **dadurch gekennzeichnet, dass** dieser durch haarnadelförmige Oligodesoxynukleotide abgeschlossen ist, zwischen denen ein Promotor am 5'-Ende und ein Terminationssignal am 3'-Ende eines DNA-Doppelstranges angeordnet ist, wobei der DNA-Doppelstrang eine 19 - 23 Basen lange, singuläre Kopie einer Gensequenz einmal in 5' - 3'-Richtung und einmal in 3' - 5'-Richtung enthält, wobei zwischen der 5' - 3' - und 3' - 5'-Orientierung der singulären Kopie der Gensequenz jeweils eine nicht selbstkomplementäre 8 - 12 Basen lange Sequenzfolge von zwei Einzelsträngen angeordnet ist, welche so gewählt sind, dass gegenüberliegende Basen in keinem Fall komplementär zueinander sind und die flankierenden Doppelstrangbereiche so durch zwei DNA-Einzelstränge miteinander verbunden sind.

## Claims

1. A method for the manufacture of vectors, which will after their transfection in eukaryotic cells inhibit specifically the formation of defined proteins via RNA interference, **characterized by** the following steps
a) partial annealing of a partially self-complimentary oligodeoxynucleotide for the formation of a DNA double strand, comprising a single copy of a 19 - 23 base pair long copy of a gene sequence in a 5'- 3' direction as well as in a 3' - 5* direction, wherein between the 5' - 3' and 3' - 5' orientated single copy of the DNA sequence a non-self complementary 8 - 12 base pair long sequence of two single strands is arranged, which are chosen in a way that opposing bases will by no means be complimentary to each other so that the flanking double stranded areas are connected via DNA single stands, wherein the DNA double strand has short protruding ends of single stranded DNA at its ends, and mixing said DNA double strand
with
hairpin loop shaped oligodeoxynucleotides having short protruding ends of single stranded DNA at their ends
and
a promoter having short protruding ends of single stranded DNA, wherein the single stranded 5' end of the promoter is able to pair with one of the hairpin loop shaped oligodeoxynucleotide and the single stranded 3' end of the promoter is complementary to the single stranded 5' end of the DNA double strand and
a termination signal for RNA polymerases with short protruding ends of single stranded DNA wherein the 5' overhang of the termination signal is able to pair specifically with the 3' end of the DNA double strand and the 3' overhang of the termination signal can specifically pair with one the hairpin loop shaped oligodeoxynucleotide and
b) subsequent ligation of the DNA fragments, as well as
c) final purification of the manufactured factors.

2. The method of claim 1, wherein the promoter is part of a bacterially amplifiable plasmid, which is cut with a restriction endonuclease prior to mixing the components in method step 1a), the restriction endonuclease recognizing a restriction site flanking the promoter, which is not present on the molecule to be produced.

3. The method of claim 2, wherein the ligation step according to 1b) is performed in the presence of the restriction endonuclease cutting the promoter out of the plasmid.

4. The method of claim 2 or 3, wherein prior to the final purification step according to 1c) a digest of the reaction mixture is performed using an endonuclease specific for 3' or 5' DNA ends.

5. The method of at least one of claims 2 to 4, wherein the restriction endonuclease is an enzyme chosen from the group of class II restriction endonucleases, preferably an enzyme from the group of BbsI, BbvI, BbVII, BpiI BplI, BsaI, BsmAI, BsmBI, BsmFI, BspMI, Eaml104I, Earl, Eco31I, Esp3I, FokI, Hgal, SfaNI or their isochizomeres.

6. The method according to one of the proceeding claims, wherein the added promoter is the promoter of the human gene for H1 RNA (sec ID. 2).

7. A method according to at least one of the proceeding claims, wherein the hairpin loop shaped oligodeoxynucleotide have in their double stranded area a recognition site for a restriction endonuclease.

8. The method according to at least one of the proceeding claims, wherein the purification in step Ic) is done by chromatography or gel electrophoresis.

9. A vector, manufactured according to at least one of claims 1 to 8, which will specifically inhibit the formation of defined proteins after its transfection in eukaryotic cells, **characterized in, that** said vector has hairpin loop-shaped oligodeoxynucleotides at its ends, having a promoter arranged at the 5'end and a termination signal at a 3' end of a DNA double strand, wherein the DNA double strand comprises a single copy of a 19 - 23 base pair long single copy of a gene sequence in a 5' - 3' direction as well as in a 3' - 5' direction, wherein between the 5' - 3' and 3' - 5' orientated single copy of the DNA sequence a non-self complementary 8 - 12 base pair long sequence of two single strands is arranged, which are chosen in a way that opposing bases will by no means be complimentary to each other so that the flanking double stranded areas are connected via DNA single strands.

## Revendications

1. Procédé de fabrication de vecteurs qui, après leur transfection dans des cellules eucaryotes, empêchent de manière sélective la formation de protéines définies au moyen d'interférence RNA, **caractérisé par** les étapes de procédé suivantes :
a) Renaturation partielle d'un oligodésoxynucléotide partiellement auto-complémentaire pour la formation d' un double brin d' ADN, qui contient une copie unique, ayant une longueur de 19 à 23 hases, d'une séquence de gène une fois dans la direction 5'-3' et une fois dans la direction 3'-5', dans lequel une séquence non-auto-complémentaire, ayant une longueur de 8 à 12 bases, de deux simples brins est disposée respectivement entre les orientations 5'-3' et 3'-5' de la copie unique de la séquence de gène, qui sont choisis de sorte que de bases opposées ne sont en aucun cas complémentaires l'une à l'autre, et les régions flanquant à double brin sont liées l'une à l'autre par deux simples brins d'ADN, dans lequel le double brin d'ADN présente aux extrémités des extrémités courtes faisant saillie d'ADN à simple brin,
et mélange du double brin d'ADN
et d'un oiigodésoxynucléotide en forme de boucle en épingle à cheveux, qui présente aux extrémités des extrémités courtes faisant saillie d'ADN à simple brin,
et d' un promoteur avec des extrémités courtes faisant saillie d'ADN à simple brin, dans lequel l'extrémité 5' à simple brin du promoteur peut former une paire avec un des oligodésoxynucléotides en forme de boucle en épingle à cheveux, et l' extrémité 3' à simple brin du promoteur est complémentaire à l'extrémité 5' à simple brin du double brin d'ADN,
et d' un signal de terminaison pour des ARN polymérases avec des extrémités courtes faisant saillie d' ADN à simple brin, dans lequel l'extrémité 5' faisant saillie du signal de terminaison peut spécifiquement former une paire avec l' extrémité 3' du double brin d'ADN, et l'extrémité 3' faisant saillie du signal de terminaison peut spécifiquement former une paire avec un oligodésoxynucléotide en forme de boucle en épingle à cheveux, et
b) ligature suivante des fragments d'ADN, et
c) purification finale des vecteurs synthétisés.

2. Procédé de fabrication selon la revendication 1, dans lequel le promoteur fait partie d'un plasmide amplifiable de bacteries, qui, avant le mélange des composants dans l'étape de procédé 1 a), est coupé au moyen d'une endonucléase de restriction, qui reconnait un site de coupure flanquant le promoteur sur le plasmide et n'étant pas compris dans la molécule destinée à être fabriquée.

3. Procédé selon la revendication 2, dans lequel l'étape de ligature selon l'étape de procédé 1 b) a lieu en présence de l'endonucléase de restriction au moyen de laquelle le promoteur a été séparé du plasmide.

4. Procédé selon l'une des revendications 2 ou 3, dans lequel avant l'étape de purification selon l'étape de procédé 1 c) une digestion du mélange de réaction a lieu au moyen d'une exonucléase spécifique exclusivement pour les extrémités d' ADN 3' ou 5'.

5. Procédé selon au moins l'une des revendications 2 à 4, dans lequel l'endonucléase de restriction est une enzyme du groupe des endonucléases de restriction de classe II, de préférence une enzyme du groupe
BbsI, BbvI, BbvII, Bpil BplI, BsaI, BsmAI, BsmBI, BsmFI, BspMI, Eaml104I, Earl, Eco31I, Esp3I, FokI, HgaI, SfaNI ou de leurs Isoschizomères.

6. Procédé selon l'une des revendications précédentes, dans lequel le promoteur ajouté est le promoteur du gène humain pour H1 RNA (SeqID.2).

7. Procédé selon au moins l'une des revendications précédentes, dans lequel les oligodesoxynucléotides en forme de boucle en épingle à cheveux présentent la séquence de reconnaissance pour une endonucléase de restriction dans leur région à double brin.

8. Procédé selon au moins l'une des revendications précédentes, dans lequel la purification dans l'étape de procédé 1 c) est réalisée par chromatographie et/ou par electrophorèse de gel.

9. Vecteur, fabriqué selon au moins l'une des revendications 1 à 8, qui, après leur transfection dans des cellules eucaryotes, empêche de manière sélective la formation de protéines définies au moyen d'interférence RNA, **caractérisé en ce que** celui est terminé par des oligedesoxynucléotides en forme de boucle en épingle à cheveux, entre lesquels est disposé un promoteur à l'extrémité 5' et un signal de terminaison à l'extrémité 3' d' un double brin d'ADN, dans lequel le double brin d' ADN contient une copie unique, ayant une longueur de 19 à 23 bases, d'une séquence de gène, une fois dans la direction 5'-3' et une fois dans la direction 3'-5', dans lequel une séquence non-auto-complémentaire, ayant une longueur de 8 à 12 bases, de deux simples brins est disposé respectivement entre l'orientation 5'-3' et celle 3'-5' de la copie unique de la séquence de gène, qui sont choisis de sorte que de bases opposées ne sont en aucun cas complémentaires l'une à l'autre, et le régions flanquant à double brin sont liées l'une a l'autre par deux simples brins d'ADN.
